(19) Europäisches Patentamt
European Patent Office
Office européen des brevets

(11) Veröffentlichungsnummer: **0 150 678**
**B1**

# (12) EUROPÄISCHE PATENTSCHRIFT

(45) Veröffentlichungstag der Patentschrift: **01.03.89**

(51) Int. Cl.⁴: **C 07 D 213/64**, A 01 N 53/00

(21) Anmeldenummer: **84810646.4**

(22) Anmeldetag: **19.12.84**

(54) **Cyclopropancarbonsäure-alpha-methyl-(6-phenoxy)-2-picolylester.**

(30) Priorität: **12.01.84 CH 140/84**
**30.11.84 CH 5713/84**

(43) Veröffentlichungstag der Anmeldung:
**07.08.85 Patentblatt 85/32**

(45) Bekanntmachung des Hinweises auf die Patenterteilung:
**01.03.89 Patentblatt 89/9**

(84) Benannte Vertragsstaaten:
**AT BE CH DE FR IT LI NL SE**

(56) Entgegenhaltungen:
**EP-A- 0 112 293**
**DE-A- 2 829 329**
**US-A- 4 221 799**
**US-A- 4 323 574**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Ackermann, Peter, Dr., Hangelimattweg 113, CH-4148 Pfeffingen (CH)**

**EP 0 150 678 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Cyclopropancarbonsäure-α-methyl-(6-phenoxy)-2-picolylester, Verfahren zu ihrer Herstellung und ihre Verwendung in der Schädlingsbekämpfung.

Die Cyclopropylcarbonsäure-α-methyl-(6-phenoxy)-2-picolylester haben die Formel

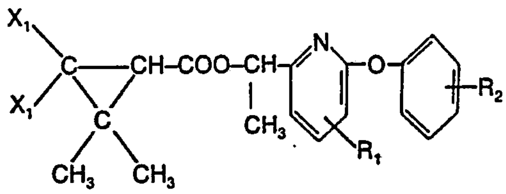

(I),

worin

$X_1$ Methyl oder Halogen,

$R_1$ Wasserstoff oder Halogen und

$R_2$ Wasserstoff, Halogen, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, $C_1$–$C_4$-Halogenalkylthio, $C_2$–$C_4$-Alkenyl, $C_2$–$C_4$-Alkinyl, Nitro oder Cyano bedeuten.

Unter Halogen ist dabei Fluor, Chlor, Brom oder Jod zu verstehen.

Die Alkyl-, Alkoxy-, Alkylthio-, Halogenalkyl, Halogenalkoxy-, Halogenalkylthio-, Alkenyl- und Alkinylgruppen können geradkettig oder verzweigt sein. Beispiele solcher Gruppen sind u.a. Methyl, Methoxy, Methylthio, Trifluormethyl, Trifluormethoxy, Aethyl, Aethoxy, Propyl, Isopropyl, n-Butyl, Vinyl, 1-Propenyl, Aethinyl, 1-Propinyl.

Besonders bevorzugt sind Verbindungen der Formel I, worin

$X_1$ Methyl oder Chlor,

$R_1$ Wasserstoff oder Halogen und

$R_2$ Wasserstoff, Halogen, $C_1$–$C_4$-Alkyl, $C_1$–$C_4$-Alkoxy, $C_1$–$C_4$-Alkylthio, Vinyl oder Aethinyl bedeuten.

Insbesondere bevorzugt sind Verbindungen der Formel I, worin

$X_1$ Methyl oder Chlor,

$R_1$ Wasserstoff und

$R_2$ Wasserstoff oder Halogen bedeuten.

Die Verbindungen der Formel I werden nach an sich bekannten Methoden z.B. wie folgt hergestellt:

1) 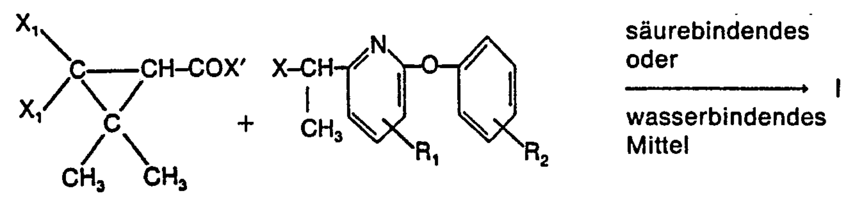


(II) (III)

2) 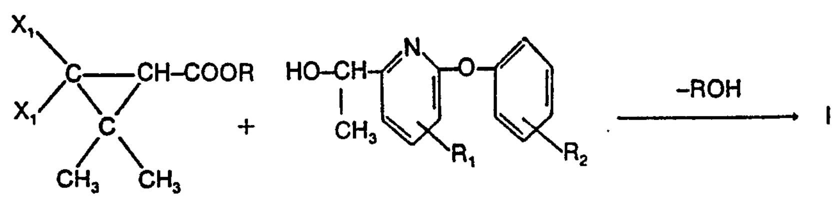


(IV) (V)

In den Formeln II bis V haben $R_1$, $R_2$ und $X_1$ die für die Formel I angegebene Bedeutung.

In den Formeln II und III steht eines der Symbole X und X' für eine Hydroxylgruppe und das andere für ein Halogenatom, insbesondere für Chlor oder Brom, oder beide Symbole für eine Hydroxylgruppe, und in der Formel IV steht R für $C_1$–$C_4$-Alkyl, insbesondere für Methyl oder Aethyl. Als säurebindende Mittel kommen insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z.B. Kalium-t.butylat und Natriummethylat in Betracht. Als wasserbindendes Mittel kann z.B. Dicyclohexylcarbodiimid verwendet werden. Die Verfahren 1 und 2 werden bei einer Reaktionstemperatur zwischen −10 und 120 °C, meist zwischen 20 und 80 °C, bei normalem oder erhöhtem Druck und vorzugsweise in einem inerten Lösungs- oder Verdünnungsmittel durchgeführt. Als Lösungs- oder Verdünnungsmittel eignen sich z.B. Äther und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis V sind bekannt oder können analog bekannten Methoden hergestellt werden.

Die Verbindungen der Formel I liegen als Gemische von verschiedenen optisch aktiven Isomeren vor, wenn bei der Herstellung nicht einheitlich optisch aktive Ausgangsmaterialien verwendet werden. Die verschiedenen Isomerengemische

können nach bekannten Methoden in die einzelnen Isomeren aufgetrennt werden. Unter der Verbindung der Formel I versteht man sowohl die einzelnen Isomeren, als auch deren Gemische.

Phenoxypicolylester der Tetramethylcyclopropancarbonsäure

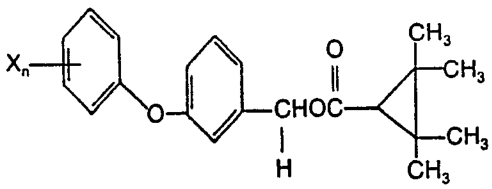

sind als Schädlingsbekämpfungsmittel aus der US-Patentschrift 4 22 799 bekannt.

Als Pestizid ist das α-Methyl-(6-phenoxy-2-pyridyl)-methyl-3-(2,2-dichlorethenyl)-2,2-dimethylcyclopropancarboxylat in der US-Patentschrift 4 323 574 beschrieben.

Die Verbindungen der Formel I eignen sich zur Bekämpfung von verschiedenartigen Schädlingen an Tieren und Pflanzen. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Vor allem eignen sich Verbindungen der Formel I zur Bekämpfung von pflanzenschädigenden Insekten, insbesondere pflanzenschädigenden Fraßinsekten, in Zier- und Nutzpflanzen, insbesondere in Baumwoll- und Reiskulturen (z.B. Spodoptera littoralis, Heliothis virescens, Nephotettix cincticeps, Nilaparvata lugens, Chilo suppressalis und Laodelphax striatellus) sowie Gemüse- und Obstkulturen (z.B. Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savignyi und Scotia ypsilon).

Wirkstoffe der Formel I zeigen auch eine sehr günstige Wirkung gegen Fliegen, wie z.B. Musca domestica sowie Mückenlarven und gegen ektoparasitäre Milben und Zecken, z.B. der Familien Ixodidae, Argasidae und Dermanyssidae. Daneben zeichnen sich die Verbindungen der Formel I durch eine breite ovizide und ovolarvizide Wirkung aus.

Die Verbindungen der Formel I weisen auch eine ausgezeichnete Wirkung gegen keratinfressende Insekten auf, wie z.B. keratinfressende Larven von Lepidoptera, z.B. Tineola spec. und Tinea spec. sowie keratinfressende Larven von Coleoptera, z.B. Anthrenus spec. und Attagenus spec. Die Wirkstoffe der Formel I eignen sich vorzüglich zum Schützen von keratinischem bzw. keratinhaltigem Material gegen Insektenfraß, insbesondere zur wasch- und lichtechten Ausrüstung gegen Insekten, insbesondere zur Motten- und Käferechtausrüstung von derartigen Materialien. Es kann keratinisches bzw. keratinhaltiges Material sowohl in rohem als auch in verarbeitetem Zustand ausgerüstet werden, z.B. rohe oder verarbeitete Schafwolle, Produkte aus anderen Tierhaaren, Felle, Pelze und Federn.

Praktisch besonders wichtig ist die Wirksamkeit der Verbindungen der Formel I gegen die Larven der Kleidermotte (Tineola bisselliela) und Pelzmotte (Tinea pelionella) sowie auch gegen die Larven der Pelz- und Teppichkäfer (Attagenus spec. bzw. Anthrenus spec.). Bevorzugt werden daher die Verbindungen der Formel I einerseits zum Schützen von Textilien aus Wolle, z.B. von Wolldecken, Wollteppichen, Wollwäsche, Wollkleidern und Wirkwaren bzw. von wollhaltigen Textilien, wie Mischgeweben, deren eine Komponente Wolle ist, z.B. Mischgewebe aus Wolle und anderen Naturfasern, vorzugsweise Baumwolle oder aus Wolle und Kunstfasern, andererseits auch zum Schützen von Pelzen und Fellen vor dem Befall durch die erwähnten Schädlinge, eingesetzt.

Die akarizide bzw. insektizide Wirkung läßt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithionat, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugte Fraktionen $C_8$ bis $C_{12}$, wie z.B. Xylolgemische oder

substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche, synthetische oberflächenaktive Verbindungen sein.

Als Seifen eignen sich die Alkali-, Erdalkali- oder gegebenenfalls substituierten Ammoniumsalze von höheren Fettsäuren ($C_1$–$C_{22}$), wie z.B. die Na- oder K-Salze der Öl- oder Stearinsäure, oder von natürlichen Fettsäuregemischen, die z.B. aus Kokosnuß- oder Tallöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entspechende Phosphate wie z.B. Salze des Phosphorsäureesters eines p-Nonylphenol-(4–14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxid-Addukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Pulblikationen beschrieben:

«McCutcheon's Detergents and Emulsifiers Annual» MC Publishing Corp., Ridgewood, New Jersey, 1979; Dr. Helmut Stache «Tensid Taschenbuch», Carl Hanser Verlag München/Wien 1981.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I
(% = Gewichtsprozent)

| 1. Emulsions-Konzentrate | a) | b) | c) |
|---|---|---|---|
| Wirkstoff der Formel I | 25% | 40% | 50% |
| Ca-Dodecylbenzolsulfonat | 5% | 8% | 6% |
| Ricinusöl-polyäthylenglykoläther (36 Mol AaO) | 5% | – | – |
| Tributylphenol-polyäthylenglykoläther (30 Mol AeO) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff der Formel I | 80% | 10% | 5% | 95% |
| Äthylenglykol-monomethyläther | 20% | – | – | – |
| Polyäthylenglykol (MG 400) | – | 70% | – | – |
| N-Methyl-2-pyrrolidon | – | 20% | – | – |
| Epoxidiertes Kokosnußöl | – | – | 1% | 5% |
| Benzin (Siedegrenzen 160–190 °C) | – | – | 94% | – |

Die Lösungen sind zur Anwendung in Form kleinster Tropfen geeignet.

| 3. Granulate | a) | b) |
|---|---|---|
| Wirkstoff der Formel I | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoff der Formel I | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) | c) |
|---|---|---|---|
| Wirkstoff der Formel I | 20% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyäthylenglykoläther (7–8 Mol AeO) | – | 2% | – |
| Hochdisperse Kieselsäure | 5% | 10% | 10% |
| Kaolin | 67% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff der Formel I | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff der Formel I | 5% | 8% |
| Talkum | 95% | – |
| Kaolin | – | 92% |

Man erhält anwendungsfertige Stäubemittel, indem der Wirkstoff mit dem Träaer vermischt und auf einer geeigneten Mühle vermahlen wird.

| 8. Extruder Granulat | |
|---|---|
| Wirkstoff der Formel I | 10% |
| Na-Ligninsulfonat | 2% |
| Carboxymethylcellulose | 1% |
| Kaolin | 87% |

Der Wirkstoff wir mit den Zusatzstoffen vermischt, vermahlen und mit Wasser angefeuchtet. Dieses Gemisch wird extrudiert und anschließend im Luftstrom getrocknet.

| 9. Umhüllungs-Granulat | |
|---|---|
| Wirkstoff der Formel I | 3% |
| Polyäthylenglykol (MG 200) | 3% |
| Kaolin | 94% |

Der feingemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

| 10. Suspensions-Konzentrat | |
|---|---|
| Wirkstoff der Formel I | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wäßrige Formaldehyd-Lösung | 0,2% |
| Silikonöl in Form einer 75%igen wäßrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung der Verbindung der Formel

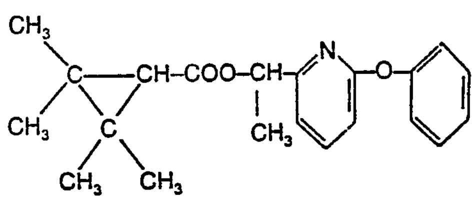

Bei 0 °C werden nacheinander zu 6,7 g der Verbindung der Formel

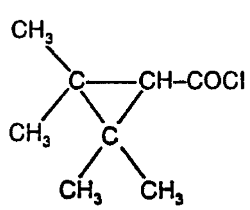

in 30 ml Toluol, 4,3 g Pyridin und 8,9 g der Verbindung der Formel

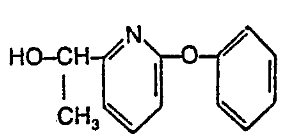

in 35 ml Toluol zugetropft. Nach 30stündigem Rühren bei 25 °C wird das Reaktionsgemisch auf 2 N Salzsäure gegossen und mit Äther extrahiert, die Ätherphase wird mit gesättigter Natriumbicarbonat- und Kochsalzlösung gewaschen, über Magnesiumsulfat getrocknet und eingeengt. Nach dem Chromatographieren des Produktes an Kieselgel mit Toluol/Essigester (95:5) erhält man die Titelverbindung Nr. 1 als Diastereomerengemisch mit einem Schmelzpunkt von 63–64 °C.

Auf analoge Weise werden auch folgende Verbindungen hergestellt:

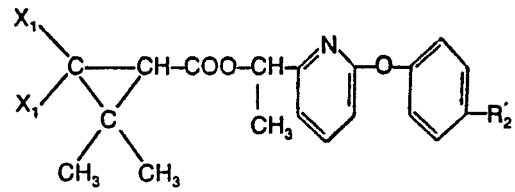

| Nr. | $X_1$ | $R_2'$ | Physikalische Daten |
|---|---|---|---|
| 2 | $CH_3$ | Cl | Smp.: 100–101 °C |
| 3 | Cl | Cl | $n_D^{20°}$ = 1,5575 |
| 4 | $CH_3$ | Br | Smp.: 87–88 °C |
| 5 | Cl | Br | $n_D^{20°}$ = 1,5686 |
| 6 | $CH_3$ | J | $n_D^{21°}$ = 1,5609 |
| 7 | Cl | J | $n_D^{20°}$ = 1,5872 |
| 8 | $CH_3$ | F | Smp.: 96 °C |
| 9 | Cl | F | Smp.: 73 °C |
| 10 | $CH_3$ | $CH_3$ | Smp.: 94–96 °C |
| 11 | $CH_3$ | $OCH_3$ | Smp.: 73–75 °C |
| 12 | Cl | $SCH_3$ | $n_D^{27°}$ = 1,5749 |
| 13 | $CH_3$ | $SCH_3$ | Smp.: 52–54 °C |
| 14 | Cl | $OCH_3$ | Smp.: 68–74 °C |
| 15 | $CH_3$ | $-CH{=}CH_2$ | Smp.: 50–52 °C |
| 16 | $CH_3$ | $-C{\equiv}CH$ | Smp.: 73–74 °C |
| 17 | $CH_3$ | H | $[\alpha]_D$ = −41° (C = 0,33; $CHCl_3$) |
| 18 | $CH_3$ | H | $[\alpha]_d$ = +34,5° (C = 0,28; $CHCl_3$) |

Beispiel 2

Insektizide Fraßgift-Wirkung: Nilaparvata lugens

Reispflanzen werden mit einer Versuchslösung, enthaltend 50 oder 100 ppm der zu prüfenden Verbindung, besprüht. Nach dem Antrocknen des Belages werden die Pflanzen mit Nymphen von Nilaparvata lugens (N3-Stadium) besetzt. Man verwendet pro Versuchsverbindung und pro Test-Spezies zwei Pflanzen. Die Auswertung der erzielten Abtötungsrate erfolgt nach 6 Tagen. Der Versuch wird bei 22 °C und 60% relativer Luftfeuchtigkeit durchgeführt.

Verbindungen gemäß Beispiel 1 zeigen im obigen Test bei 50 ppm Wirkstoffkonzentration 100% Wirkung gegenüber Nilaparvata lugens-Nymphen.

Beispiel 3

Wirkung gegen Diabrotica balteata

750 ml sandige Erde werden mit 150 ml Testlösung enthaltend 3; 0,75; 0,2 oder 0,05 ppm Wirkstoff gemischt. Mit der behandelten Erde werden Maiskeimlinge in Plastiktöpfe eingetopft (4 Keimlinge pro Topf mit einem Durchmesser von 10 cm). Die Töpfe werden unmittelbar nachher mit 10 $L_3$-Larven von Diabrotica balteata infestiert. Die Kontrolle wird 10 Tage nach dem Einsetzen der Larven durchgeführt.

Verbindungen gemäß Beispiel 1 zeigen im obigen Test bei 3 ppm Wirkstoffkonzentration 100%ige Wirkung gegen $L_3$-Larven von Diabrotica balteata.

Beispiel 4

Wirkung gegen Zecken

A) Amblyomma hebraeum

50 Nymphen werden in ein Glasröhrchen gezählt und für 1 bis 2 Minuten in 2 ml einer wäßrigen Emulsion aus einer Verdünnungsreihe mit je 10, 1 oder 0,1 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Auswertung erfolgt nach 1 Woche. Für jeden Versuch werden 2 Wiederholungen durchgeführt.

B) Boophilus microplus (Larven)

Mit einer analogen Verdünnungsreihe wie beim Test A) werden mit je 20 sensiblen resp. OP-resistenten Larven Versuche durchgeführt. (Die Resistenz bezieht sich auf die Verträglichkeit von Diazinon).

Verbindungen gemäß dem Herstellungsbeispiel 1 wirken bei 0,1 ppm Wirkstoffkonzentration 100%ig gegen Nymphen bzw. Larven der Zecken: Amblyomma hebraeum und Boophilus microplus.

Beispiel 5

Insektizide Fraßgift-Wirkung: Spodoptera littoralis

Baumwollpflanzen werden mit einer Versuchslösung, enthaltend 100, 200 oder 400 ppm der zu prüfenden Verbindung, besprüht.

Nach dem Antrocknen des Belages werden die Pflanzen mit Larven von Spodoptera littoralis (L1-Stadium) besetzt. Man verwendet pro Versuchsberbindung und pro Test-Spezies zwei Pflanzen. Die Auswertung der erzielten Abtötungsrate erfolgt nach 2, 4, 24 und 48 Stunden. Der Versuch wird bei 28 °C und 60% relativer Luftfeuchtigkeit durchgeführt.

Verbindungen gemäß Beispiel 1 zeigen im obigen Test bei 400 ppm Wirkstoffkonzentration 100%ige Wirkung gegenüber Spodoptera littoralis-Larven.

Beispiel 6

Wirkung gegen die Kleidermotte, den Pelz- und Teppichkäfer

a) Ausziehmethode

Es wird jeweils eine 0,4%ige Stammlösung einer der Verbindungen gemäß Beispiel 1 in Methylcellosolve hergestellt. Dann wird bei Zimmertemperatur eine wäßrige Applikationsflotte hergestellt, die in 120 ml destilliertem Wasser 0,12 ml «Sandozin KB®», 0,6 ml Ameisensäure 1:10 und 0,75 ml der jeweiligen 0,4%igen Stammlösung enthält. Dann werden 3 g Wollflanell-Gewebe mit heißem Wasser durchgenetzt und bei Zimmertemperatur eingegeben. Unter ständigem Umziehen des Wollmusters wird die Badtemperatur innerhalb 20 Minuten auf 60 °C erwärmt und 30 Minuten bei 60 °C behandelt. Dann wird abgekühlt, das Wollmuster zweimal 3 Minuten mit destilliertem Wasser gespült, von Hand abgequetscht und an der Luft getrocknet. Die Wirkstoffkonzentration beträgt 1000 ppm, berechnet auf das Wollgewicht.

Das so getrocknete Muster wird der Prüfung gegen die Larven der Kleidermotte (Tineola bisselliella Hum.), des Pelzkäfers Attagenus piceus und Teppichkäfers (Anthrenus vorax Wat.) unterworfen.

Aus den behandelten Wollflanellmustern werden Stücke gleicher Grösse ausgeschnitten und 14 Tage lang bei konstanter Temperatur (28 °C) und konstanter relativer Luftfeuchtigkeit (65%) dem Angriff (Fraß) von je 15 Larven des entsprechenden Schädlings ausgesetzt. Die Beurteilung erfolgt einerseits nach dem relativen Gewichtsverlust des Prüflings und andererseits nach der Anzahl noch lebender Organismen.

Die geprüften Verbindungen gemäß Beispiel 1 zeigen eine 100%ige Wirkung gegen die 3 verwendeten Schädlinge.

b) Foulardmethode

Es wird jeweils eine 0,4%ige Stammlösung einer der Verbindungen gemäß Beispiel 1 in Methylcellosolve bereitet. 12,5 ml der jeweiligen Stammlösung werden mit Methylcellosolve, welche 0,65 g/l «Sandozin KB®» enthält, auf 50 ml verdünnt (= Lösung Nr. 1). 25 ml der Lösung Nr. 1 werden mit Methylcellosolve, welche 0,5 g/l «Sandozin KB®» enthält, auf 50 ml verdünnt (= Lösung Nr. 2). 25 ml von Lösung Nr. 2 werden erneut mit Methylcellosolve, welche 0,5 g/l «Sandozin KB®» enthält, auf 50 ml verdünnt (= Lösung Nr. 3).

Von den Lösungen Nr. 1, 2 und 3 werden je 3 ml in Kristallisierschalen geleert und je eine geköderte Rondelle aus Wollflanell 3 Sekunden darin benetzt. Die feuchten Rondellen werden anschließend zwischen Aluminiumfolien foulardiert, und zwar derart, daß die abgequetschten Rondellen je 50% Flotte aufgenommen haben. Die Konzentrationen an Wirkstoff sind dann der Reihe nach 500 ppm, 250 ppm und 125 ppm für behandelte Rondellen aus den Lösungen Nr. 1, 2 und 3.

Die feuchten Rondellen werden an der Luft getrocknet und den gleichen biologischen Prüfungen unterworfen, wie im Beispiel 6a) beschrieben.

Die geprüften Verbindungen gemäß Beispiel 1 zeigen eine 100%ige Wirkung gegen alle 3 Schädlinge, auch bei der geringsten Konzentation von 125 ppm.

## Patentansprüche

1. Ein Cyclopropancarbonsäure-α-methyl-(6-phenoxy)-2-picolylester der Formel

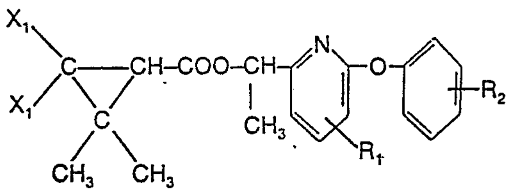

(I),

worin

$X_1$ Methyl oder Halogen,

$R_1$ Wasserstoff oder Halogen und

$R_2$ Wasserstoff, Halogen, $C_1$–$C_4$-Alkyl, $C_1$–$C_4$-Alkoxy, $C_1$–$C_4$-Alkylthio, $C_1$–$C_4$-Halogenalkyl, $C_1$-$C_4$-Halogenalkoxy, $C_1$-$C_4$-Halogenalkylthio, $C_2$–$C_4$-Alkenyl, $C_2$–$C_4$-Alkinyl, Nitro oder Cyano bedeuten.

2. Eine Verbindung gemäß Anspruch 1, worin

$X_1$ Methyl oder Chlor,

$R_1$ Wasserstoff oder Halogen und

$R_2$ Wasserstoff, Halogen, $C_1$–$C_4$-Alkyl, $C_1$–$C_4$-Alkoxy, $C_1$–$C_4$-Alkylthio, Vinyl oder Aethinyl bedeuten.

3. Eine Verbindung gemäß Anspruch 2, worin

$X_1$ Methyl oder Chlor,

$R_1$ Wasserstoff und

$R_2$ Wasserstoff oder Halogen bedeuten.

4. Die Verbindung gemäß Anspruch 3 der Formel

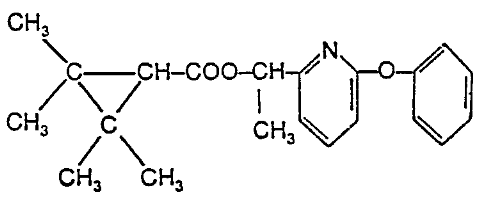

5. Die Verbindung gemäß Anspruch 3 der Formel

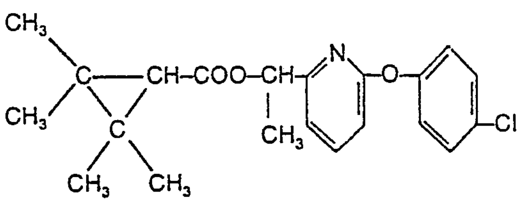

.

6. Die Verbindung gemäß Anspruch 3 der Formel

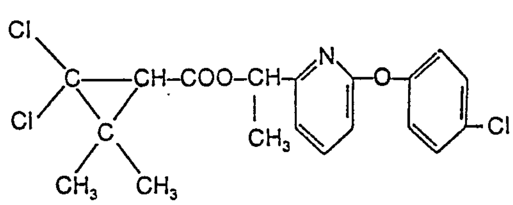

.

7. Die Verbindung gemäß Anspruch 3 der Formel

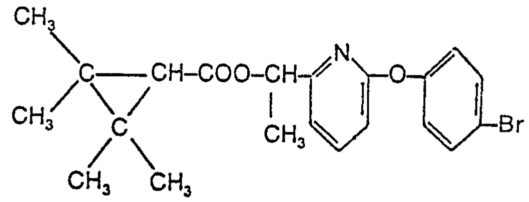

.

8. Die Verbindung gemäß Anspruch 3 der Formel

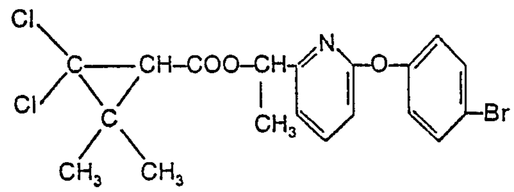

.

9. Die Verbindung gemäß Anspruch 3 der Formel

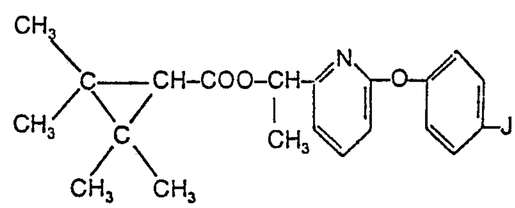

.

10. Die Verbindung gemäß Anspruch 3 der Formel

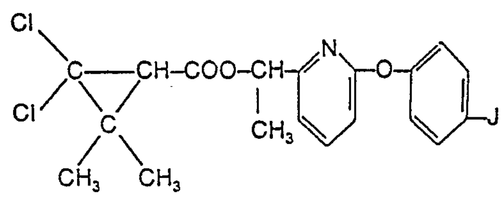

.

11. Die Verbindung gemäß Anspruch 3 der Formel

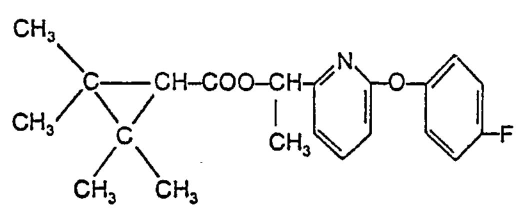

.

12. Die Verbindung gemäß Anspruch 3 der Formel

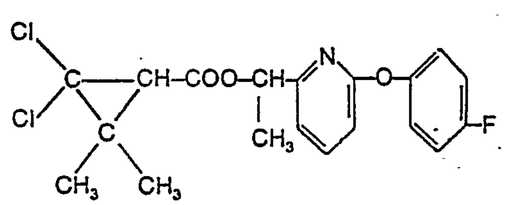

.

13. Die Verbindung gemäß Anspruch 2 der Formel

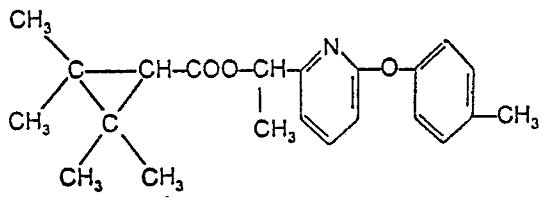 .

14. Die Verbindung gemäß Anspruch 2 der Formel

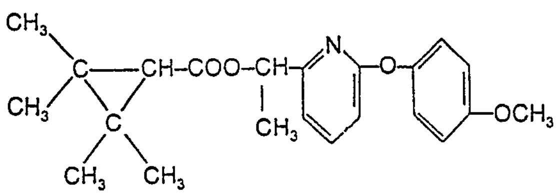 .

15. Die Verbindung gemäß Anspruch 2 der Formel

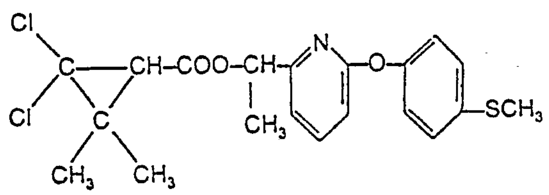 .

16. Die Verbindung gemäß Anspruch 2 der Formel

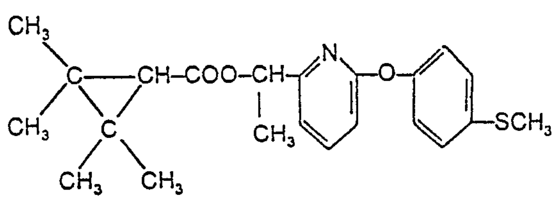 .

17. Die Verbindung gemäß Anspruch 2 der Formel

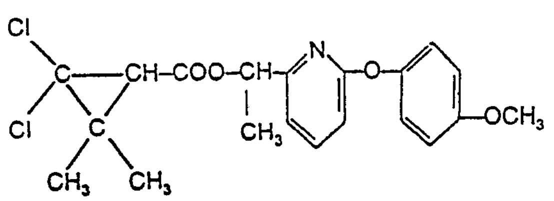 .

18. Die Verbindung gemäß Anspruch 2 der Formel

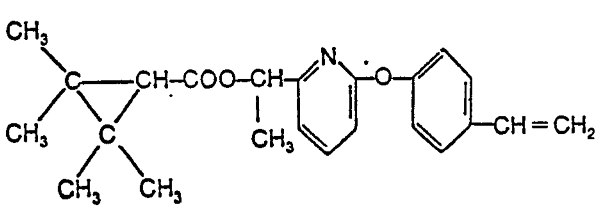 .

19. Die Verbindung gemäß Anspruch 2 der Formel

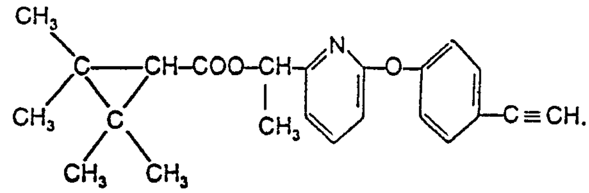

20. Verfahren zur Herstellung von Verbindungen gemäß Anspruch 1, dadurch gekennzeichnet, daß man eine Verbindung der Formel

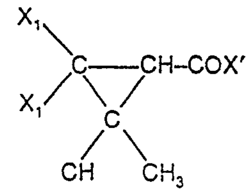

in Gegenwart eines säurebindenden Mittels mit einer Verbindung der Formel

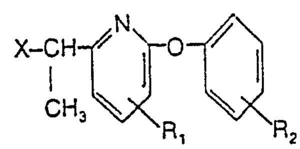

umsetzt, worin $R_1$, $R_2$ und $X_1$ die im Anspruch 1 angegebene Bedeutung haben und eines der Symbole X′ und X für eine Hydroxylgruppe und das andere für ein Halogenatom steht.

21. Ein Schädlingsbekämpfungsmittel, welches als aktive Komponente eine Verbindung gemäß Anspruch 1 und in der Formulierungstechnik übliche Hilfsmittel enthält.

## Claims

1. An α-methyl-(6-phenoxy)-2-picolyl cyclopropanecarboxylate of the formula

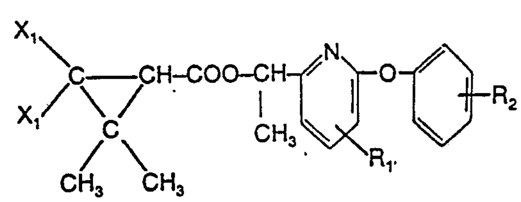 (I),

wherein

$X_1$ is methyl or halogen,

$R_1$ is hydrogen or halogen, and

$R_2$ is hydrogen, halogen, $C_1$–$C_4$alkyl, $C_1$–$C_4$alkoxy, $C_1$–$C_4$alkylthio, $C_1$–$C_4$haloalkyl, $C_1$–$C_4$haloalkoxy, $C_1$–$C_4$haloalkylthio, $C_2$–$C_4$alkenyl, $C_2$–$C_4$alkynyl, nitro or cyano.

2. A compound according to claim 1, wherein

$X_1$ is methyl or chlorine,

$R_1$ is hydrogen or halogen, and

$R_2$ is hydrogen, halogen, $C_1$–$C_4$alkyl, $C_1$–$C_4$alkoxy, $C_1$–$C_4$alkylthio, vinyl or ethynyl.

3. A compound according to claim 2, wherein
$X_1$ is methyl or chlorine,
$R_1$ is hydrogen, and
$R_2$ is hydrogen or halogen.

4. The compound according to claim 3 of the formula

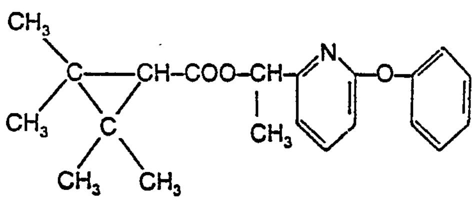

5. The compound according to claim 3 of the formula

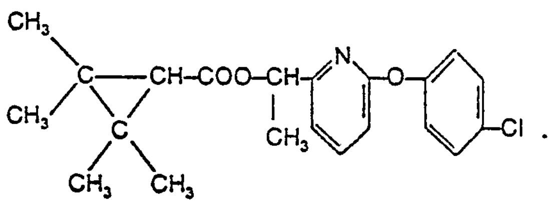

6. The compound according to claim 3 of the formula

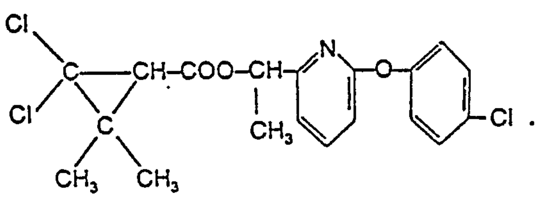

7. The compound according to claim 3 of the formula

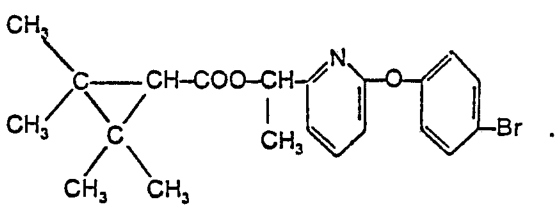

8. The compound according to claim 3 of the formula

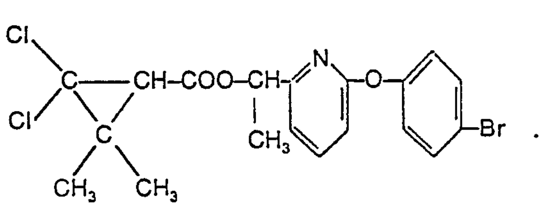

9. The compound according to claim 3 of the formula

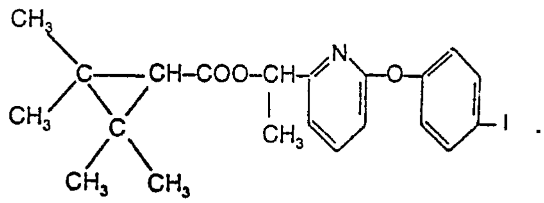

10. The compound according to claim 3 of the formula

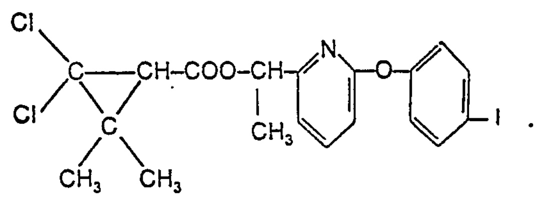

11. The compound according to claim 3 of the formula

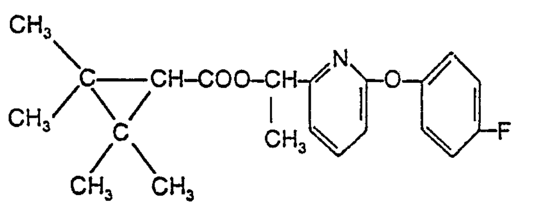

12. The compound according to claim 3 of the formula

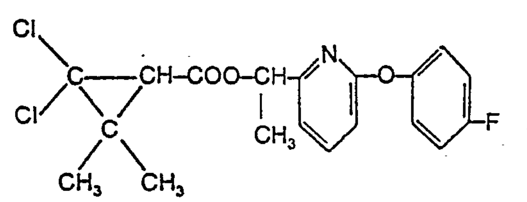

13. The compound according to claim 2 of the formula

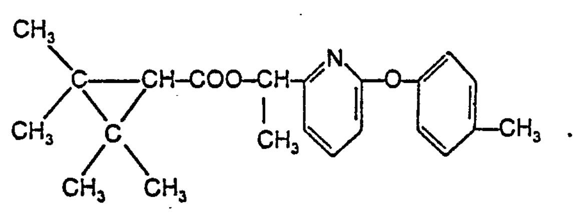

14. The compound according to claim 2 of the formula

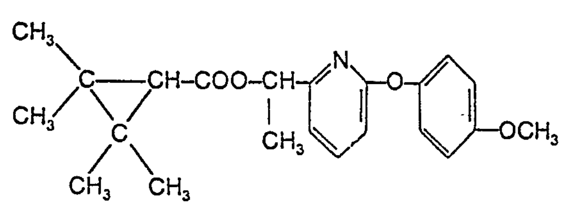

15. The compound according to claim 2 of the formula

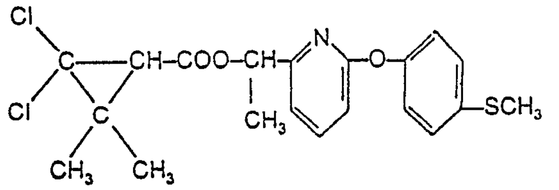 .

16. The compound according to claim 2 of the formula

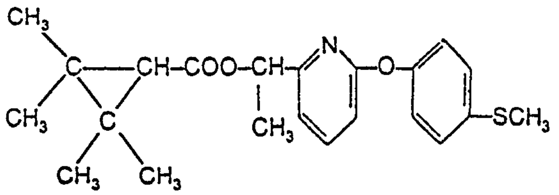 .

17. The compound according to claim 2 of the formula

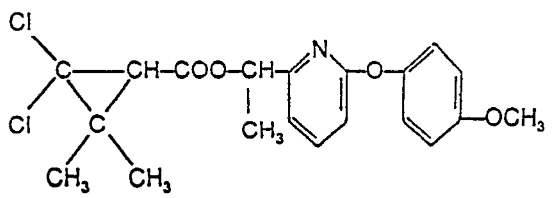 .

18. The compound according to claim 2 of the formula

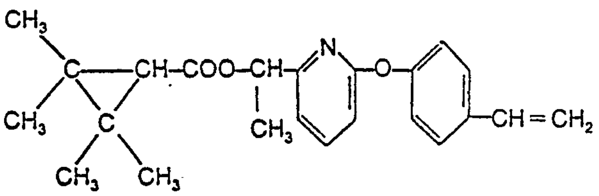 .

19. The compound according to claim 2 of the formula

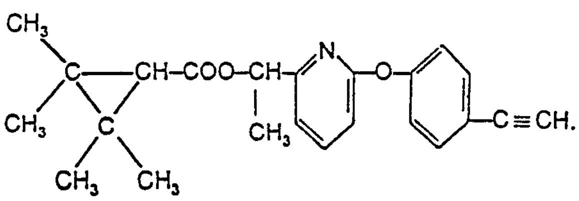

20. A process for the preparation of compounds according to claim 1, which comprises reacting a compound of the formula

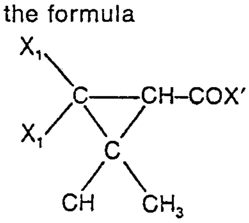

with a compound of the formula

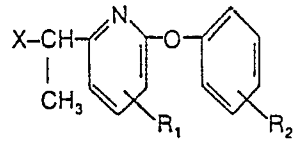

wherein $R_1$, $R_2$ and $X_1$ are as defined in claim 1 and one of the symbols X' and X is a hydroxyl group and the other is a halogen atom, in the presence of an acid acceptor.

21. A pesticidal composition which contains, as active ingredient, a compound according to claim 1, and adjuvants conventionally employed in the art of formulation.

**Revendications**

1. Un ester alpha-méthyl-(6-phénoxy)-2-picolylique d'acide cyclopropane-carboxylique de formule

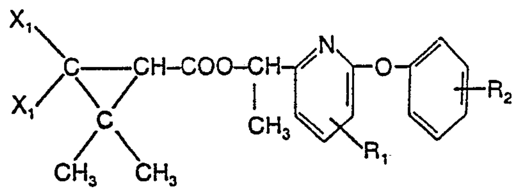 (I),

dans laquelle

$X_1$ représente un groupe méthyle ou un halogène,

$R_1$ représente l'hydrogène ou un halogène et

$R_2$ représente l'hydrogène, un halogène, un groupe alkyle en $C_1$–$C_4$, alcoxy en $C_1$–$C_4$, alkylthio en $C_1$–$C_4$, halogénoalkyle en $C_1$–$C_4$, halogénoalcoxy en $C_1$–$C_4$, halogénoalkylthio en $C_1$–$C_4$, alcényle en $C_2$–$C_4$, alcynyle en $C_2$–$C_4$, nitro ou cyano.

2. Un composé selon la revendication 1, dans lequel

$X_1$ représente un groupe méthyle ou le chlore,

$R_1$ représente l'hydrogène ou un halogène et

$R_2$ représente l'hydrogène, un halogène, un groupe alkyle en $C_1$–$C_4$, alcoxy en $C_1$–$C_4$, alkylthio en $C_1$–$C_4$, vinyle ou éthynyle.

3. Un composé selon la revendication 2, dans lequel

$X_1$ représente un groupe méthyle ou le chlore,

$R_1$ représente l'hydrogène et

$R_2$ représente l'hydrogène ou un halogène.

4. Le composé selon la revendication 3, de formule:

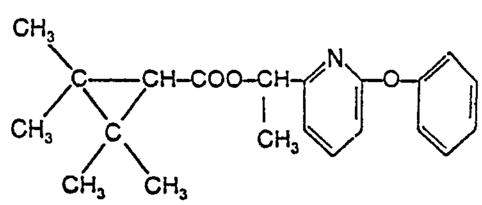

5. Le composé selon la revendication 3, de formule:

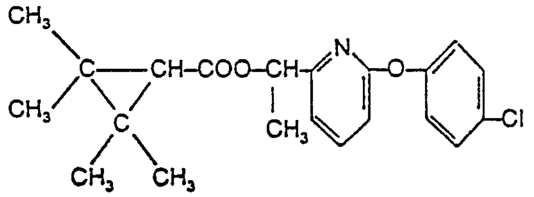 .

6. Le composé selon la revendication 3, de formule:

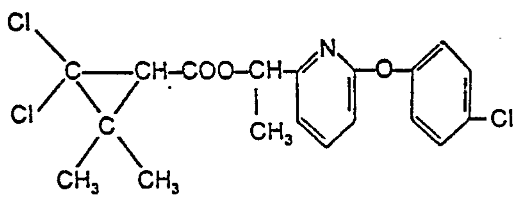 .

7. Le composé selon la revendication 3, de formule:

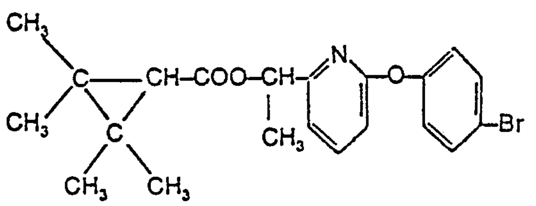 .

8. Le composé selon la revendication 3, de formule:

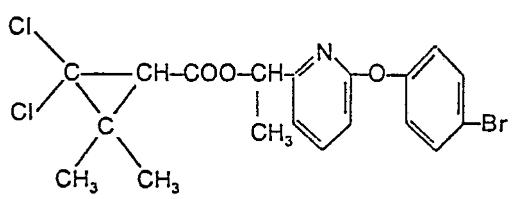 .

9. Le composé selon la revendication 3, de formule:

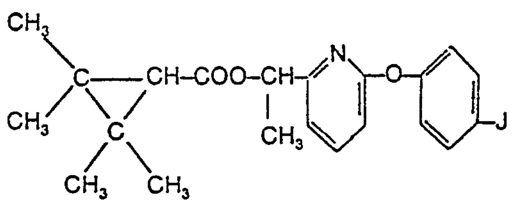 .

10. Le composé selon la revendication 3, de formule:

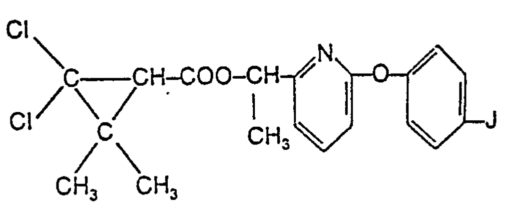 .

11. Le composé selon la revendication 3, de formule:

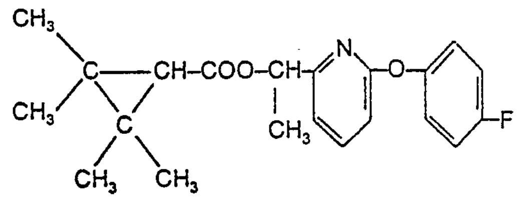 .

12. Le composé selon la revendication 3, de formule:

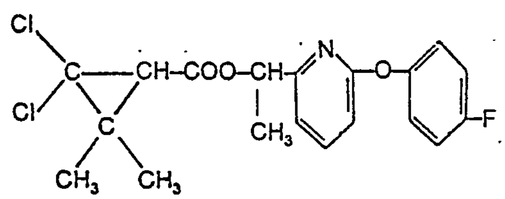 .

13. Le composé selon la revendication 2, de formule:

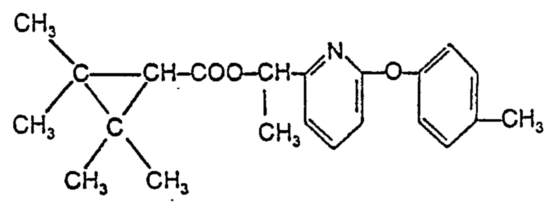 .

14. Le composé selon la revendication 2, de formule:

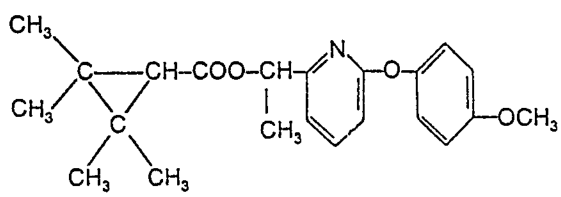 .

15. Le composé selon la revendication 2, de formule:

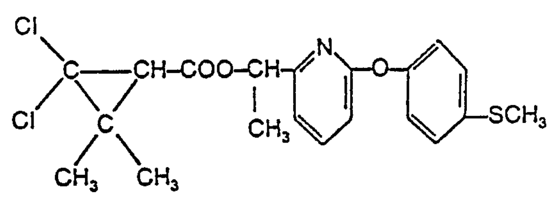 .

16. Le composé selon la revendication 2, de formule:

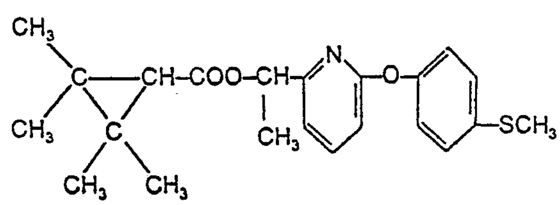 .

17. Le composé selon la revendication 2, de formule:

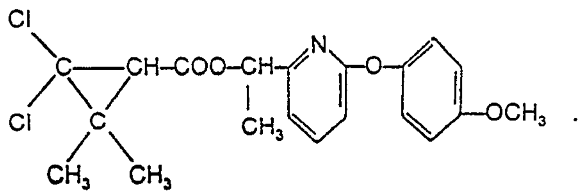

18. Le composé selon la revendication 2, de formule:

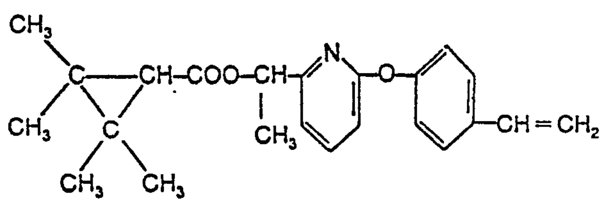

19. Le composé selon la revendication 2, de formule:

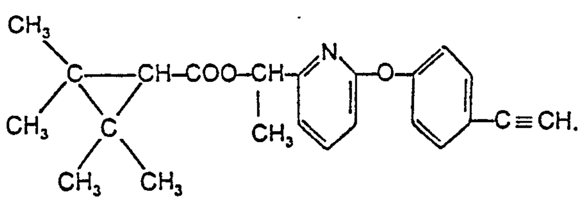

20. Procédé de préparation des composés selon la revendication 1, caractérisé en ce que l'on fait réagir un composé de formule

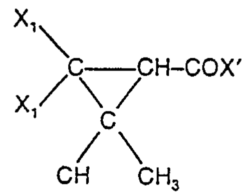

en présence d'un accepteur d'acide, avec un composé de formule

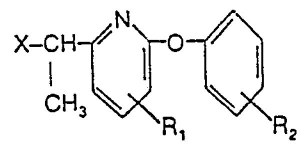

$R_1$, $R_2$ et $X_1$ ayant les significations indiquées dans la revendication 1, l'un des symboles X' et X représentant un groupe hydroxy et l'autre un atome d'halogène.

21. Un produit pesticide contenant, en tant que composant actif, un composé selon la revendication 1, et des produits auxiliaires usuels dans les techniques de formulation.